(19) Europäisches Patentamt / European Patent Office / Office européen des brevets

(11) **EP 4 035 594 B1**

(12) **EUROPEAN PATENT SPECIFICATION**

(45) Date of publication and mention
of the grant of the patent:
**11.03.2026 Bulletin 2026/11**

(21) Application number: **20867064.6**

(22) Date of filing: **09.09.2020**

(51) International Patent Classification (IPC):
*A61B 5/02* *(2006.01)*   *A61B 5/024* *(2006.01)*
*A61B 5/11* *(2006.01)*   *A61B 5/16* *(2006.01)*

(52) Cooperative Patent Classification (CPC):
**A61B 5/4812;** A61B 5/024; A61B 5/1118

(86) International application number:
**PCT/JP2020/034179**

(87) International publication number:
**WO 2021/059988 (01.04.2021 Gazette 2021/13)**

(54) **SLEEP STAGE ESTIMATION DEVICE, SLEEP STAGE ESTIMATION METHOD, AND PROGRAM**

SCHLAFPHASEN-SCHÄTZVORRICHTUNG, SCHLAFPHASEN-SCHÄTZVERFAHREN UND -PROGRAMM

DISPOSITIF D'ESTIMATION DE STADE DU SOMMEIL, PROCÉDÉ D'ESTIMATION DE STADE DU SOMMEIL, ET PROGRAMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.09.2019 JP 2019173326**

(43) Date of publication of application:
**03.08.2022 Bulletin 2022/31**

(73) Proprietor: **Casio Computer Co., Ltd.
Tokyo 151-8543 (JP)**

(72) Inventors:
• **MAENO, Yasushi**
  **Hamura-shi, Tokyo 205-8555 (JP)**
• **UEDA, Masashi**
  **Hamura-shi, Tokyo 205-8555 (JP)**
• **HAMADA, Akira**
  **Hamura-shi, Tokyo 205-8555 (JP)**
• **NAKAGOME, Kouichi**
  **Hamura-shi, Tokyo 205-8555 (JP)**
• **NAKAJIMA, Mitsuyasu**
  **Hamura-shi, Tokyo 205-8555 (JP)**
• **YAMAYA, Takashi**
  **Hamura-shi, Tokyo 205-8555 (JP)**

(74) Representative: **Grünecker Patent- und
Rechtsanwälte
PartG mbB
Leopoldstraße 4
80802 München (DE)**

(56) References cited:
JP-A- 2004 089 267   JP-A- 2004 089 267
JP-A- 2004 254 827   JP-A- 2004 254 827
JP-A- 2016 067 811   JP-A- 2016 067 811
JP-A- 2017 169 884   JP-A- 2017 221 413
JP-A- 2017 221 413   JP-A- 2019 048 150
JP-A- 2019 048 150   US-A1- 2017 273 617

• FONSECA PEDRO ET AL: "Cardiorespiratory
Sleep Stage Detection Using Conditional
Random Fields", IEEE JOURNAL OF
BIOMEDICAL AND HEALTH INFORMATICS, IEEE,
PISCATAWAY, NJ, USA, vol. 21, no. 4, 1 July 2017
(2017-07-01), pages 956 - 966, XP011655139,
ISSN: 2168-2194, [retrieved on 20170629], DOI:
10.1109/JBHI.2016.2550104

EP 4 035 594 B1

**Description**

TECHNICAL FIELD

The present invention relates to sleep state monitoring technology.

BACKGROUND ART

As a sleep state monitoring system for determining a sleep state, JP-A-2011-83393 widely uses a determination of sleep stages (light sleep, deep sleep, REM sleep, non-REM sleep, and the like) by brain waves. In addition, JP-A-2018-161432 also suggests a sleep monitoring system using body movements and pulse waves, which are easier to obtain than brain waves.

Further, the article "Cardiorespiratory Sleep State Detection Using Conditional Random Fields" by Pedro Fonseca et al., IEEE Journal of biomedical and health informatics, vol. 21, no. 4, 1 July 2017, gives an overview about cardiorespiratory sleep state detection using conditional random fields, wherein the usage of conditional random field models is an example of discriminated models for sleep state classification is compared to other kinds of models such as hidden Markov models. A plurality of data is acquired from the subject, including electrocardiogram (ECG) and thoracic respiratory inductance plethysmography (RIP) data.

SUMMARY OF INVENTION

The present invention provides a sleep stage estimation device, a sleep stage estimation method and a non-transitory computer-readable medium as defined in the independent claims.

BRIEF DESCRIPTION OF DRAWINGS

[FIG. 1] FIG. 1 is a schematic view showing a sleep stage estimation device according to an embodiment of the present disclosure.
[FIG. 2] FIG. 2 is a block diagram showing a hardware configuration of the sleep stage estimation device according to the embodiment of the present disclosure.
[FIG. 3] FIG. 3 is a block diagram showing a functional configuration of the sleep stage estimation device according to the embodiment of the present disclosure.
[FIG. 4] FIG. 4 is a flowchart showing sleep stage estimation processing unit according to an embodiment of the present disclosure.

DESCRIPTION OF EMBODIMENTS

In a following embodiment, a sleep stage estimation device configured to estimate a sleep stage sequence from time-series data during sleep of a subject is disclosed.

[Outline of Present Disclosure]

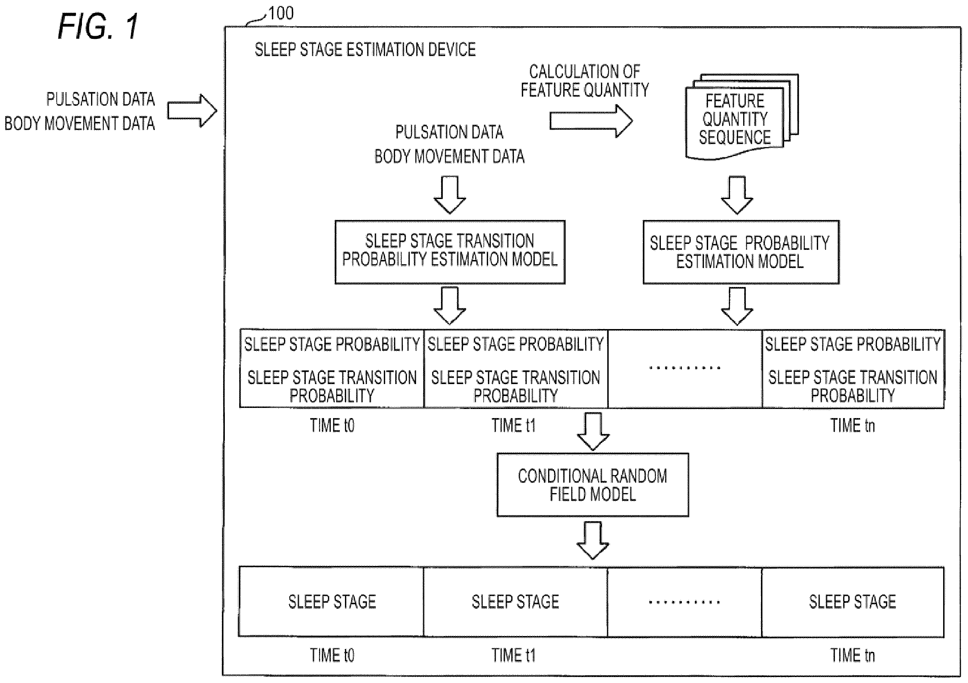

An embodiment described below is outlined. As shown in FIG. 1, when pulsation data such as a pulse wave and a heartbeat during sleep of a subject and body movement data such as an acceleration and an angular velocity are acquired, a sleep stage estimation device 100 calculates a feature quantity (for example, an average, a standard deviation, an LF/HF ratio and the like of heartbeat intervals) every predetermined time from the pulsation data, and generates a feature quantity sequence from the feature quantities obtained in time series. The sleep stage estimation device 100 is configured to estimate a sleep stage probability, which indicates a probability that the subject is estimated to be in each sleep stage, from the feature quantity at each time by using a learned sleep stage probability estimation model. For example, the sleep stage probability estimation model may be implemented by a logistic regression model, and may also be implemented by a multi-class logistic regression model when classifying a sleep stage into three or more sleep stages (for example, light sleep, deep sleep, REM sleep, non-REM sleep, and the like).

On the other hand, the sleep stage estimation device 100 is configured to calculate body movement information by calculating an angular velocity of rotation at each time from the body movement data at each time. Then, by using a learned sleep stage transition probability estimation model, a sleep stage transition probability, which considers a time series showing a probability that the subject is estimated to have transitioned from one sleep stage to another sleep stage, is estimated from the body movement information. For example, the sleep stage transition probability estimation model may be implemented by a sigmoid function.

The sleep stage estimation device 100 is configured to estimate a sleep stage sequence from the estimated sleep stage probability and sleep stage transition probability at each time by using a learned conditional random field (CRF) model. The conditional random field model is implemented by a feature function in which the sleep stage probability is an observation feature and the sleep stage transition probability is a transition feature.

In this way, the sleep stage estimation device 100 can estimate the sleep stage sequence of the subject from the pulsation data and body movement data collected in time series from the sleeping subject by sensors attached to the subject.

[Hardware Configuration]

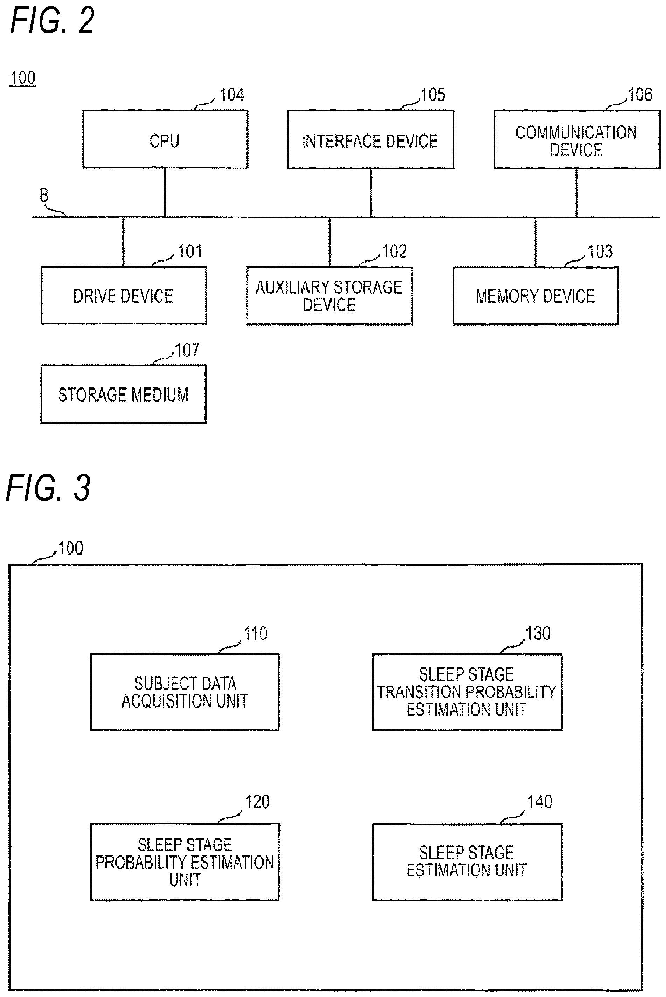

Here, the sleep stage estimation device 100 may be, for example, a computing device such as a server, or may have a hardware configuration as shown in FIG. 2. That is, the sleep stage estimation device 100 includes a drive device 101, an auxiliary storage device 102, a memory device 103, a CPU (Central Processing Unit) 104, an interface device 105, and a communication device 106, which are interconnected via a bus B.

A variety of computer programs including programs or instructions for implementing various functions and processing, which will be described later, in the sleep stage estimation device 100 may be provided by a recording medium 107 such as a CD-ROM (Compact Disk-Read Only Memory). When the recording medium 107 having the program recorded thereon is set in the drive device 101, the program is installed into the auxiliary storage device 102 from the recording medium 107 via the drive device 101. However, the program does not necessarily have to be installed by the recording medium 107, and may be downloaded from any one external device via a network or the like. The auxiliary storage device 102 is configured to store the installed program and also to store necessary files, data and the like.

The memory device 103 is configured to read and store the program or data from the auxiliary storage device 102 when an instruction to start the program is issued. The auxiliary storage device 102 and the memory device 103 are implemented as a non-transitory computer-readable storage medium for storing a program or an instruction. The CPU 104 functioning as a processor is configured to execute a variety of functions and processing of the sleep stage estimation device 100, according to the program and various data such as parameters necessary for executing the program, the program and the data being stored in the memory device 103. The interface device 105 is used as a communication interface for connecting to a network or an external device. The communication device 106 is configured to execute a variety of communication processing for communicating with the external device.

However, the sleep stage estimation device 100 is not limited to the above-described hardware configuration. For example, the sleep stage estimation device 100 may also be implemented by any other suitable hardware configuration such as one or more circuits configured to implement one or more of the functions and processing by the sleep stage estimation device 100. For example, the sleep stage estimation device 100 may be implemented as a wristwatch-type wearable type device that is worn on an arm of the subject or an earphone-type hearable type device that is inserted into an ear of the subject.

[Sleep Stage Estimation Device]

Next, the sleep stage estimation device 100 according to an embodiment of the present disclosure is described with reference to FIG. 3. FIG. 3 is a block diagram showing a functional configuration of the sleep stage estimation device 100 according to the embodiment of the present disclosure.

As shown in FIG. 3, the sleep stage estimation device 100 includes a subject data acquisition unit 110, a sleep stage probability estimation unit 120, a sleep stage transition probability estimation unit 130, and a sleep stage estimation unit 140.

The subject data acquisition unit 110 is configured to acquire pulsation data and body movement data of a subject. For example, the pulsation data may be collected by a pulse wave sensor or a heartbeat sensor worn on an arm, an ear or the like of the sleeping subject. For example, the body movement data may be collected by an acceleration sensor, a body movement sensor or the like configured to detect body movement with being worn on the arm, the ear or the like of the sleeping subject, like the pulsation data.

The sleep stage estimation device 100 may be connected to the sensor in a wired or wireless manner, and the subject data acquisition unit 110 may be configured to acquire the collected pulsation data and body movement data via a communication line. Alternatively, the collected pulsation data and body movement data may be transmitted and stored in a computing device (not shown) connected to the sensor in a wired or wireless manner, and the subject data acquisition unit 110 may be configured to acquire the pulsation data and body movement data from the computing device. The subject data acquisition unit 110 is configured to provide the acquired pulsation data and/or body movement data to the sleep stage probability estimation unit 120 and the sleep stage transition probability estimation unit 130.

The sleep stage probability estimation unit 120 is configured to calculate (acquire) a feature quantity sequence from the pulsation data, and to estimate a sleep stage probability sequence of the subject from the calculated (acquired) feature quantity sequence by using a learned sleep stage probability estimation model. Specifically, when the pulsation data is acquired from the subject data acquisition unit 110, the sleep stage probability estimation unit 120 calculates a feature quantity $f_{ti}$ (i=0, 1, $\cdots$, n) indicative of any one of an average, a standard deviation, an LF / HF ratio and the like of heartbeat intervals every predetermined time (for example, every 30 seconds, every 1 minute, every 5 minutes, or the like) from a heartbeat interval sequence of the pulsation data, and generates a feature quantity sequence $f_{t0}$, $f_{t1}$, $\cdots$, $f_{tn}$ from each calculated feature quantity $f_{ti}$. Then, the sleep stage probability estimation unit 120 inputs each calculated feature quantity $f_{ti}$ to the learned sleep stage probability estimation model, estimates a sleep stage probability $P_{stage\_ti}$ of each sleep stage for the feature quantity $f_{ti}$ at time ti, and provides the sleep stage estimation unit 140 with a sleep stage probability sequence $P_{max\_stage\_t0}$, $P_{max\_stage\_t1}$, $\cdots$, $P_{max\_stage\_tn}$ of a sleep stage probability $P_{max\_stage\_ti}$ that a probability $P_{stage\_ti}$ for the feature quantity $f_{ti}$ at time ti is greatest.

In one embodiment, the learned sleep stage probability estimation model may be configured by a logistic regression model that estimates the probability of each sleep stage from the feature quantities of the feature quantity sequence. As for the logistic regression model, for example, when a sleep stage set S is {$s_0$(=light sleep), $s_1$(= deep sleep), $s_2$(=REM sleep) and $s_3$(=non-REM sleep)}, a multi-class logistic regression model may be used. That is, training data consisting of a pair of the feature quantity such as the average, the standard deviation, the LF/HF ratio and the like of the heartbeat interval at each time and a correct sleep stage determined from brain wave data at that time is prepared in advance. Supervised learning using the training data is executed on the multi-class logistic regression model such as any suitable logistic curve. The multi-class logistic regression model can be learned according to any well-known learning method pertaining to learning of the multi-class logistic regression model, and parameters such as a weight vector of the multi-class logistic regression are updated so that the correct sleep stage can be correctly predicted for the feature quantity of the training data. For example, a cross entropy of probabilities of the training data and the correct answer data may be obtained, and a parameter that minimizes the value of the cross entropy may be obtained. Specifically, a method such as a stochastic gradient descent method may be used to repeatedly update the parameters by using partial differential values of each parameter of the cross entropy and to obtain a parameter that minimizes the cross entropy. Note that, in practice, it is possible to perform learning by using a function of any appropriate library.

The sleep stage transition probability estimation unit 130 is configured to calculate (acquire) a body movement amount sequence from the body movement data, and to estimate a sleep stage transition probability sequence of the subject from the calculated (acquired) body movement amount sequence by using the learned sleep stage transition probability estimation model. Specifically, when the body movement data is acquired from the subject data acquisition unit 110, the sleep stage transition probability estimation unit 130 calculates a body movement amount $m_{ti}$ (i=0, 1, $\cdots$, n) such as an angular velocity every predetermined time (for example, every 30 seconds, every 1 minute, every 5 minutes, or the like) from the body movement data, and generates a body movement amount sequence $m_{t0}$, $m_{t1}$, $\cdots$, $m_{tn}$ from each calculated body movement amount $f_{ti}$. Then, the sleep stage transition probability estimation unit 130 inputs each body movement amount $m_{ti}$ to the learned sleep stage transition probability estimation model, estimates a sleep stage transition probability $P_{transition\_ti}$ for the body movement amount $m_{ti}$ at time ti, and provides the sleep stage estimation unit 140 with the estimated sleep stage transition probability sequence $P_{transition\_t0}$, $P_{transition\_t1}$, $\cdots$, $P_{transition\_tn}$.

In one embodiment, the learned sleep stage transition probability estimation model may be configured by a sigmoid function that estimates a transition probability between sleep stages from the body movement amounts of the body movement amount sequence. For example, the sigmoid function may be formulated as follows.

$$\Pr(m_{ti}) = 1/(1 + \exp(-ax + b))$$

Note that, the parameters a and b may be determined at the time of learning ta conditional random field (CRF) model, which will be described later. Note that, the parameters of the sleep stage transition probability estimation model may be learned at the same time as the learning of the conditional random field (CRF), which will be described later, or may be learned as a part of the parameters of the conditional random field (CRF).

The sleep stage estimation unit 140 estimates a sleep stage sequence of the subject from the sleep stage probability sequence and the sleep stage transition probability sequence by using the learned conditional random field model. Specifically, when the sleep stage probability sequence $P_{max\_stage\_t0}$, $P_{max\_stage\_t1}$, $\cdots$, $P_{max\_stage\_tn}$ and the sleep stage transition probability sequence $P_{transition\_t0}$, $P_{transition\_t1}$, $\cdots$, $P_{transition\_tn}$ are respectively acquired from the sleep stage probability estimation unit 120 and the sleep stage transition probability estimation unit 130, the sleep stage estimation unit 140 inputs the sleep stage probability $P_{max\_stage\_ti}$ and the sleep stage transition probability $P_{transition\_ti}$ at each time point to the learned conditional random field (CRF) model, as an observation feature and a transition feature, respectively, estimates a sleep stage $S_{ti}$ at each time, and generates a sleep stage sequence $S_{t0}$, $S_{t1}$, $\cdots$, $S_{tn}$.

In the present invention, the learned conditional random field model is configured by a feature function in which the sleep stage probability is an observation feature and the sleep stage transition probability is a transition feature. The sleep stage estimation unit 140 estimates the sleep stage $S_{ti}$ from the sleep stage probability $P_{max\_stage\_ti}$ and the sleep stage transition probability $P_{transition\_ti}$ at each time point by using the feature function. For example, a cross entropy of probabilities of learning sequence data and a correct label sequence may be obtained, and a parameter that minimizes the value of the cross entropy may be obtained. Specifically, a method such as a stochastic gradient descent method and a dynamic programming may be used to repeatedly update the parameters by using partial differential values of each parameter of the cross entropy and to obtain a parameter that minimizes the cross entropy. Note that, in practice, it is possible to perform learning by using a function of any appropriate library.

Specifically, in a case where $\lambda$ is a parameter of the conditional random field and a and b are parameters of the sleep stage transition probability, the parameters $\lambda$, a and b that minimize the cross entropy $E(\lambda, a, b)$ are learned at the same time. That is, by using values obtained by partially differentiating the cross entropy E with respect to the respective parameters $\lambda$, a and b, the parameters are repeatedly updated by a method such as a stochastic gradient descent method, and the parameters $\lambda$, a and b that minimize the cross entropy E are obtained.

For example, N learning data can be expressed as follows.

[Equation 1]

$$D\left(x^{(i)}, y^{(i)}\right)_{i=1}^{N}$$

On the other hand, the cross entropy is as follows.

[Equation 2]

$$E(\lambda, a, b) = -\sum_{i=1}^{N} l\left(x^{(i)}, y^{(i)}\right)$$

However, the logarithmic likelihood l(x, y) is as follows, where M is the length of the time series.

[Equation 3]

$$l(x, y) = \sum_{m=1}^{M} l_m(x_m, y_{m-1}, y_m)$$

[Equation 4]

$$l_m(x_m, y_{m-1}, y_m) =$$
$$\lambda^T f(x_m, y_{m-1}, y_m, a, b) - log \sum_{y' \in Y} exp(\lambda^T f(x_m, y'_{m-1}, y'_m, a, b))$$

Here, the feature function is expressed as follows.

[Equation 5]

$$f(x_m, y_{m-1}, y_m, a, b)$$

For example, the feature function consists of a sleep stage probability (K), a constant 1 and a sleep stage transition probability $p_r$, and is as follows.

[Equation 6]

$$f(x_m, y_{m-1}, y_m, a, b) = [0,0,\cdots\quad 0,\cdots,0,\underbrace{p_1, p_2, \cdots, p_K}_{\text{stage probability appears from } (y_m \times K)\text{th}}, 0,\cdots\quad, 0, \underbrace{1, p_r}_{\text{constant}}]^T$$

$$K \times K + 2$$

However, the sleep stage transition probability $p_r$ of the body movement amount q is as follows.

[Equation 7]

$$p_r(q, y_{m-1}, y_m, a, b) = \begin{cases} 1/(1 + exp(aq + b)) & (y_{m-1} = y_m) \\ 1 - 1/(1 + exp(aq + b)) & (y_{m-1} \neq y_m) \end{cases}$$

In a case of $x_m = [p_1, p_2, p_3, p_4, q]$, $y_{m-1}=3$ and $y_m=2$, for example, the feature function becomes as follows.

[Equation 8]

$$f(x_m, y_{m-1}, y_m, a, b) = [0,0,0,0,p_1, p_2, p_3, p_4, 0,0,0,0,0,0,0,0,1,1 - 1/(1 + exp(aq + b))]^T$$

[Sleep Stage Estimation Processing]

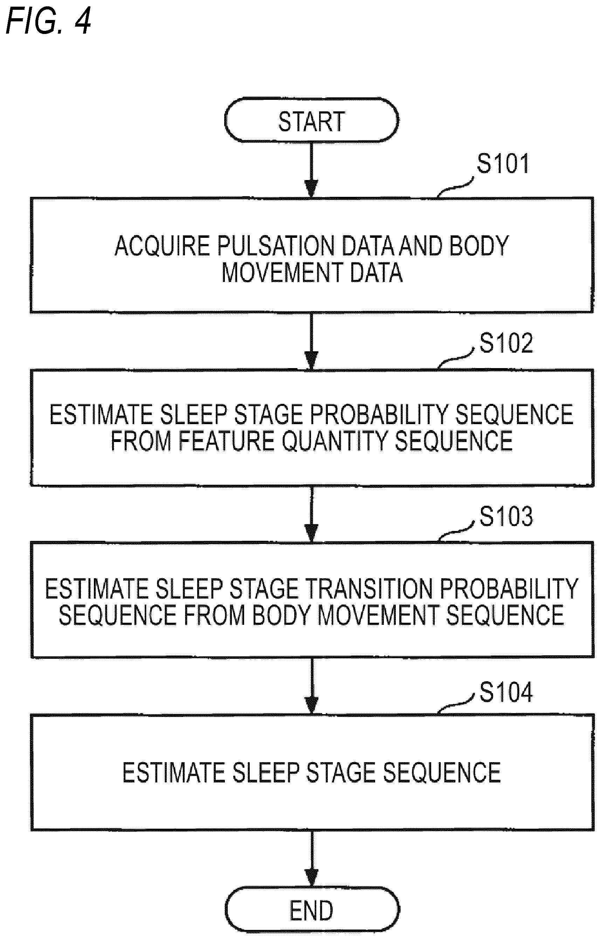

Next, sleep stage estimation processing according to an embodiment of the present disclosure is described with reference to FIG. 4. The sleep stage estimation processing is implemented by the above-described sleep stage estimation device 100, and may be implemented, for example, by the processor of the sleep stage estimation device 100 executing a program or an instruction. FIG. 4 is a flowchart showing sleep stage estimation processing according to an embodiment of the present disclosure.

As shown in FIG. 4, in step S101, the sleep stage estimation device 100 acquires pulsation data and body movement data. The pulsation data and the body movement data are collected by the above-described pulse wave sensor or the heartbeat sensor, and the acceleration sensor or the body movement sensor.

In step S102, the sleep stage estimation device 100 estimates a sleep stage probability sequence from a feature quantity sequence. Specifically, the sleep stage estimation device 100 calculates features such as an average, a standard deviation and an LF/HF ratio of heartbeat intervals every predetermined time, and inputs the calculated features to the learned sleep stage probability estimation model. The sleep stage estimation device 100 acquires a sleep stage probability from the learned sleep stage probability estimation model for each time, and generates a sleep stage probability sequence.

In step S103, the sleep stage estimation device 100 estimates a sleep stage transition probability sequence from a feature quantity sequence. Specifically, the sleep stage estimation device 100 calculates a body movement amount such as an angular velocity every predetermined time, and inputs the calculated body movement amount to the learned sleep stage transition probability estimation model. The sleep stage estimation device 100 acquires a sleep stage transition probability from the learned sleep stage transition probability estimation model for each time, and generates a sleep stage transition probability sequence.

In step S104, the sleep stage estimation device 100 estimates a sleep stage sequence. Specifically, the sleep stage estimation device 100 inputs the sleep stage probability and the sleep stage transition probability at each time to the learned sleep stage estimation model, as an observation feature and a transition feature, respectively, and estimates a sleep stage at each time, from the sleep stage probability sequence generated in step S102 and the sleep stage transition probability sequence generated in step S103. When the sleep stages are estimated at all times, the sleep stage estimation device 100 generates a sleep stage sequence.

The present invention is not limited to the above-described embodiment, and can be variously modified at the implementation stage without departing from the gist of the present invention. **In** addition, each embodiment may be implemented in combination as appropriate as possible, in which case the combined effects are obtained. Further, the above-described embodiment includes inventions at various stages, and various inventions can be extracted by an appropriate combination in a plurality of disclosed constitutional requirements. For example, even if some constitutional requirements are deleted from all the constitutional requirements shown in the embodiment, the problems described in the parts of the problems to be solved by the invention can be solved, and the configuration in which the constitutional requirements are deleted can be extracted as an invention when the effects described in the part of the effect of the invention are obtained.

The present application is based on Japanese Patent Application No. 2019-173326 filed on September 24, 2019.

**Claims**

1.  A sleep stage estimation device (100) comprising:

    a subject data acquisition unit (110) configured to acquire pulsation data and body movement data of a subject;
    a sleep stage probability estimation unit (120) configured to acquire a feature quantity sequence from the pulsation data and to estimate a sleep stage probability sequence of the subject from the acquired feature quantity sequence by using a learned sleep stage probability estimation model;
    a sleep stage transition probability estimation unit (130) configured to acquire a body movement amount sequence from the body movement data and to estimate a sleep stage transition probability sequence of the subject from the acquired body movement amount sequence by using a learned sleep stage transition probability estimation model; and
    a sleep stage estimation unit (140) configured to estimate a sleep stage sequence of the subject from the sleep stage probability sequence and the sleep stage transition probability sequence by using a learned conditional random field model,
    wherein the learned conditional random field model is configured by a feature function in which a sleep stage probability is an observation feature and a sleep stage transition probability is a transition feature.

2.  The sleep stage estimation device (100) according to claim 1, wherein the feature quantity sequence comprises one or more time-series data of an average, a standard deviation and an LF/HF ratio of heartbeat intervals every predetermined time.

3.  The sleep stage estimation device (100) according to claim 1 or 2, wherein the learned sleep stage probability estimation model is configured by a logistic regression model that estimates a probability of each sleep stage from feature quantities of the feature quantity sequence.

4.  The sleep stage estimation device (100) according to any one of claims 1 to 3, wherein the learned sleep stage probability estimation model is acquired by supervised learning using training data consisting of a pair of a feature quantity of the pulsation data and a corresponding correct sleep stage.

5.  The sleep stage estimation device (100) according to any one of claims 1 to 4, wherein the body movement amount sequence comprises time-series data of an angular velocity.

6.  The sleep stage estimation device (100) according to any one of claims 1 to 5, wherein the learned sleep stage transition probability estimation model is configured by a sigmoid function that estimates a transition probability between sleep stages from body movement amounts of the body movement amount sequence.

7.  A sleep stage estimation method that is executed by a sleep stage estimation device (100), the sleep stage estimation method comprising:

acquiring pulsation data and body movement data of a subject;

acquiring a feature quantity sequence from the pulsation data and estimating a sleep stage probability sequence of the subject from the acquired feature quantity sequence by using a learned sleep stage probability estimation model;

acquiring a body movement amount sequence from the body movement data and estimating a sleep stage transition probability sequence of the subject from the acquired body movement amount sequence by using a learned sleep stage transition probability estimation model; and

estimating a sleep stage sequence of the subject from the sleep stage probability sequence and the sleep stage transition probability sequence by using a learned conditional random field model,

wherein the learned conditional random field model is configured by a feature function in which a sleep stage probability is an observation feature and a sleep stage transition probability is a transition feature.

8. A non-transitory computer-readable storage medium that stores a computer-executable program comprises instructions which, when executed by a computer of a sleep stage estimation device (100), cause the computer to execute processing of:

acquiring pulsation data and body movement data of a subject;

acquiring a feature quantity sequence from the pulsation data and estimating a sleep stage probability sequence of the subject from the acquired feature quantity sequence by using a learned sleep stage probability estimation model;

acquiring a body movement amount sequence from the body movement data and estimating a sleep stage transition probability sequence of the subject from the acquired body movement amount sequence by using a learned sleep stage transition probability estimation model; and

estimating a sleep stage sequence of the subject from the sleep stage probability sequence and the sleep stage transition probability sequence by using a learned conditional random field model,

wherein the learned conditional random field model is configured by a feature function in which a sleep stage probability is an observation feature and a sleep stage transition probability is a transition feature.

**Patentansprüche**

1. Schlafphasen-Schätzvorrichtung (100), die Folgendes umfasst:

eine Probandendaten-Erfassungseinheit (110), die dazu konfiguriert ist, Pulsationsdaten und Körperbewegungsdaten eines Probanden zu erfassen;

eine Schlafphasen-Wahrscheinlichkeitsschätzeinheit (120), die dazu konfiguriert ist, eine Merkmalsmengensequenz aus den Pulsationsdaten zu erfassen und eine Schlafphasen-Wahrscheinlichkeitssequenz des Probanden aus der erfassten Merkmalsmengensequenz unter Verwendung eines gelernten Schlafphasen-Wahrscheinlichkeitsschätzmodells zu schätzen;

eine Schlafphasen-Übergangswahrscheinlichkeitsschätzeinheit (130), die dazu konfiguriert ist, eine Körperbewegungsmengensequenz aus den Körperbewegungsdaten zu erfassen und eine Schlafphasen-Übergangswahrscheinlichkeitssequenz des Probanden aus der erfassten Körperbewegungsmengensequenz unter Verwendung eines gelernten Schlafphasen-Übergangswahrscheinlichkeitsschätzmodells zu schätzen; und

eine Schlafphasen-Schätzeinheit (140), die dazu konfiguriert ist, eine Schlafphasensequenz des Probanden aus der Schlafphasen-Wahrscheinlichkeitssequenz und der Schlafphasen-Übergangswahrscheinlichkeitssequenz unter Verwendung eines gelernten bedingten Zufallsfeldmodells zu schätzen,

wobei das gelernte bedingte Zufallsfeldmodell durch eine Merkmalsfunktion konfiguriert ist, in der eine Schlafphasenwahrscheinlichkeit ein Beobachtungsmerkmal und eine Schlafphasen-Übergangswahrscheinlichkeit ein Übergangsmerkmal ist.

2. Schlafphasen-Schätzvorrichtung (100) nach Anspruch 1, wobei die Merkmalsmengensequenz eine oder mehrere Zeitreihendaten eines Durchschnitts, einer Standardabweichung und eines LF/HF-Verhältnisses von Herzschlagintervallen zu jeder vorgegebenen Zeit umfasst.

3. Schlafphasen-Schätzvorrichtung (100) nach Anspruch 1 oder 2, wobei das gelernte Schlafphasen-Wahrscheinlichkeitsschätzmodell durch ein logistisches Regressionsmodell konfiguriert ist, das eine Wahrscheinlichkeit jeder Schlafphase aus den Merkmalsmengen der Merkmalsmengensequenz schätzt.

**4.** Schlafphasen-Schätzvorrichtung (100) nach einem der Ansprüche 1 bis 3, wobei das gelernte Schlafphasen-Wahrscheinlichkeitsschätzmodell durch überwachtes Lernen unter Verwendung von Trainingsdaten, die aus einem Paar aus einer Merkmalsmenge der Pulsationsdaten und einer entsprechenden korrekten Schlafphase bestehen, erfasst wird.

**5.** Schlafphasen-Schätzvorrichtung (100) nach einem der Ansprüche 1 bis 4, wobei die Körperbewegungsmengensequenz Zeitreihendaten einer Winkelgeschwindigkeit umfasst.

**6.** Schlafphasen-Schätzvorrichtung (100) nach einem der Ansprüche 1 bis 5, wobei das gelernte Schlafphasen-Übergangswahrscheinlichkeitsschätzmodell durch eine Sigmoidfunktion konfiguriert ist, die eine Übergangswahrscheinlichkeit zwischen Schlafphasen anhand der Körperbewegungsmengen der Körperbewegungsmengensequenz schätzt.

**7.** Schlafphasen-Schätzverfahren, das von einer Schlafphasen-Schätzvorrichtung (100) ausgeführt wird, wobei das Schlafphasen-Schätzverfahren Folgendes umfasst:

Erfassen von Pulsationsdaten und Körperbewegungsdaten eines Probanden;
Erfassen einer Merkmalsmengensequenz aus den Pulsationsdaten und Schätzen einer Schlafphasen-Wahrscheinlichkeitssequenz des Probanden aus der erfassten Merkmalsmengensequenz unter Verwendung eines gelernten Schlafphasen-Wahrscheinlichkeitsschätzmodells;
Erfassen einer Körperbewegungsmengensequenz aus den Körperbewegungsdaten und Schätzen einer Schlafphasen-Übergangswahrscheinlichkeitssequenz des Probanden aus der erfassten Körperbewegungsmengensequenz unter Verwendung eines gelernten Schlafphasen-Übergangswahrscheinlichkeitsschätzmodells; und
Schätzen einer Schlafphasensequenz des Probanden aus der Schlafphasen-Wahrscheinlichkeitssequenz und der Schlafphasen-Übergangswahrscheinlichkeitssequenz unter Verwendung eines gelernten bedingten Zufallsfeldmodells,
wobei das gelernte bedingte Zufallsfeldmodell durch eine Merkmalsfunktion konfiguriert ist, in der eine Schlafphasenwahrscheinlichkeit ein Beobachtungsmerkmal und eine Schlafphasen-Übergangswahrscheinlichkeit ein Übergangsmerkmal ist.

**8.** Nicht transitorisches, computerlesbares Speichermedium, das ein computerausführbares Programm speichert, das Anweisungen umfasst, die, wenn sie von einem Computer einer Schlafphasen-Schätzvorrichtung (100) ausgeführt werden, den Computer veranlassen, die Verarbeitung von Folgendem auszuführen:

Erfassen von Pulsationsdaten und Körperbewegungsdaten eines Probanden;
Erfassen einer Merkmalsmengensequenz aus den Pulsationsdaten und Schätzen einer Schlafphasen-Wahrscheinlichkeitssequenz des Probanden aus der erfassten Merkmalsmengensequenz unter Verwendung eines gelernten Schlafphasen-Wahrscheinlichkeitsschätzmodells;
Erfassen einer Körperbewegungsmengensequenz aus den Körperbewegungsdaten und Schätzen einer Schlafphasen-Übergangswahrscheinlichkeitssequenz des Probanden aus der erfassten Körperbewegungsmengensequenz unter Verwendung eines gelernten Schlafphasen-Übergangswahrscheinlichkeitsschätzmodells; und
Schätzen einer Schlafphasensequenz des Probanden aus der Schlafphasen-Wahrscheinlichkeitssequenz und der Schlafphasen-Übergangswahrscheinlichkeitssequenz unter Verwendung eines gelernten bedingten Zufallsfeldmodells,
wobei das gelernte bedingte Zufallsfeldmodell durch eine Merkmalsfunktion konfiguriert ist, in der eine Schlafphasenwahrscheinlichkeit ein Beobachtungsmerkmal und eine Schlafphasen-Übergangswahrscheinlichkeit ein Übergangsmerkmal ist.

**Revendications**

**1.** Dispositif d'estimation de stade de sommeil (100) comprenant :

une unité d'acquisition de données d'un sujet (110) configurée pour acquérir des données de pouls et des données de mouvement du corps d'un sujet ;
une unité d'estimation de probabilité de stade de sommeil (120) configurée pour acquérir une séquence de quantités de caractéristique à partir des données de pouls et pour estimer une séquence de probabilités de stade de sommeil du sujet à partir de la séquence de quantités de caractéristique acquise en utilisant un modèle appris d'estimation de probabilité de stade de sommeil ;

une unité d'estimation de probabilité de transition de stade de sommeil (130) configurée pour acquérir une séquence de quantités de mouvement du corps à partir des données de mouvement du corps et pour estimer une séquence de probabilités de transition de stade de sommeil du sujet à partir de la séquence de quantités de mouvement du corps acquise en utilisant un modèle appris d'estimation de probabilité de transition de stade de sommeil ; et

une unité d'estimation de stade de sommeil (140) configurée pour estimer une séquence de stades de sommeil du sujet à partir de la séquence de probabilités de stade de sommeil et de la séquence de probabilités de transition de stade de sommeil en utilisant un modèle appris de champ aléatoire conditionnel,

dans lequel le modèle appris de champ aléatoire conditionnel est configuré par une fonction de caractéristique dans laquelle une probabilité de stade de sommeil est une caractéristique d'observation et une probabilité de transition de stade de sommeil est une caractéristique de transition.

2. Dispositif d'estimation de stade de sommeil (100) selon la revendication 1, dans lequel la séquence de quantités de caractéristique comprend une ou plusieurs données de séries temporelles d'une moyenne, d'un écart-type et d'un ratio LF/HF d'intervalles de battements cardiaques à chaque moment prédéterminé.

3. Dispositif d'estimation de stade de sommeil (100) selon la revendication 1 ou 2, dans lequel le modèle appris d'estimation de probabilité de stade de sommeil est configuré par un modèle de régression logistique qui estime une probabilité de chaque stade de sommeil à partir de quantités de caractéristique de la séquence de quantités de caractéristique.

4. Dispositif d'estimation de stade de sommeil (100) selon l'une quelconque des revendications 1 à 3, dans lequel le modèle appris d'estimation de probabilité de stade de sommeil est acquis par des données d'apprentissage utilisant un apprentissage supervisé constitué d'une paire d'une quantité de caractéristique de données de pouls et d'un stade de sommeil correct correspondant.

5. Dispositif d'estimation de stade de sommeil (100) selon l'une quelconque des revendications 1 à 4, dans lequel la séquence de quantités de mouvement du corps comprend des données de séries temporelles d'une vitesse angulaire.

6. Dispositif d'estimation de stade de sommeil (100) selon l'une quelconque des revendications 1 à 5, dans lequel le modèle appris d'estimation de probabilité de transition de stade de sommeil est configuré par une fonction sigmoïde qui estime une probabilité de transition entre des stades de sommeil à partir des quantités de mouvement du corps de la séquence de quantités de mouvement du corps.

7. Procédé d'estimation de stade de sommeil qui est exécuté par un dispositif d'estimation de stade de sommeil (100), le procédé d'estimation de stade de sommeil comprenant :

l'acquisition de données de pouls et de données de mouvement du corps d'un sujet ;

l'acquisition d'une séquence de quantités de caractéristique à partir des données de pouls et l'estimation d'une séquence de probabilités de stade de sommeil du sujet à partir de la séquence de quantités de caractéristique acquise en utilisant un modèle appris d'estimation de probabilité de stade de sommeil ;

l'acquisition d'une séquence de quantités de mouvement du corps à partir des données de mouvement du corps et l'estimation d'une séquence de probabilités de transition de stade de sommeil du sujet à partir de la séquence de quantités de mouvement du corps acquise en utilisant un modèle appris d'estimation de probabilité de transition de stade de sommeil ; et

l'estimation d'une séquence de stades de sommeil du sujet à partir de la séquence de probabilités de stade de sommeil et de la séquence de probabilités de transition de stade de sommeil en utilisant un modèle appris de champ aléatoire conditionnel,

dans lequel le modèle appris de champ aléatoire conditionnel est configuré par une fonction de caractéristique dans laquelle une probabilité de stade de sommeil est une caractéristique d'observation et une probabilité de transition de stade de sommeil est une caractéristique de transition.

8. Support de stockage lisible par ordinateur non transitoire qui stocke un programme exécutable par un ordinateur comprenant des instructions qui, lorsqu'exécutées par un ordinateur d'un dispositif d'estimation de stade de sommeil (100), amènent l'ordinateur à exécuter le traitement consistant à :

acquérir des données de pouls et des données de mouvement du corps d'un sujet ;

acquérir une séquence de quantités de caractéristique à partir des données de pouls et estimer une séquence de probabilités de stade de sommeil du sujet à partir de la séquence de quantités de caractéristique acquise en utilisant un modèle appris d'estimation de probabilité de stade de sommeil ;

acquérir une séquence de quantités de mouvement du corps à partir des données de mouvement du corps et estimer une séquence de probabilités de transition de stade de sommeil du sujet à partir de la séquence de quantités de mouvement du corps acquise en utilisant un modèle appris d'estimation de probabilité de transition de stade de sommeil ; et

estimer une séquence de stades de sommeil du sujet à partir de la séquence de probabilités de stade de sommeil et de la séquence de probabilités de transition de stade de sommeil en utilisant un modèle appris de champ aléatoire conditionnel,

dans lequel le modèle appris de champ aléatoire conditionnel est configuré par une fonction de caractéristique dans laquelle une probabilité de stade de sommeil est une caractéristique d'observation et une probabilité de transition de stade de sommeil est une caractéristique de transition.

FIG. 1

SLEEP STAGE ESTIMATION DEVICE

PULSATION DATA
BODY MOVEMENT DATA

CALCULATION OF
FEATURE QUANTITY

FEATURE QUANTITY SEQUENCE

PULSATION DATA
BODY MOVEMENT DATA

SLEEP STAGE TRANSITION PROBABILITY ESTIMATION MODEL

SLEEP STAGE PROBABILITY ESTIMATION MODEL

| SLEEP STAGE PROBABILITY | SLEEP STAGE PROBABILITY | .......... | SLEEP STAGE PROBABILITY |
| SLEEP STAGE TRANSITION PROBABILITY | SLEEP STAGE TRANSITION PROBABILITY | | SLEEP STAGE TRANSITION PROBABILITY |
| TIME t0 | TIME t1 | | TIME tn |

CONDITIONAL RANDOM FIELD MODEL

| SLEEP STAGE | SLEEP STAGE | .......... | SLEEP STAGE |
| TIME t0 | TIME t1 | | TIME tn |

EP 4 035 594 B1

12

## FIG. 2

100

| | | |
|---|---|---|
| CPU ⌒104 | INTERFACE DEVICE ⌒105 | COMMUNICATION DEVICE ⌒106 |

B

| | | |
|---|---|---|
| DRIVE DEVICE ⌒101 | AUXILIARY STORAGE DEVICE ⌒102 | MEMORY DEVICE ⌒103 |

STORAGE MEDIUM ⌒107

## FIG. 3

100

| | |
|---|---|
| SUBJECT DATA ACQUISITION UNIT ⌒110 | SLEEP STAGE TRANSITION PROBABILITY ESTIMATION UNIT ⌒130 |
| SLEEP STAGE PROBABILITY ESTIMATION UNIT ⌒120 | SLEEP STAGE ESTIMATION UNIT ⌒140 |

## FIG. 4

```
                    ┌─────────────┐
                    │    START    │
                    └──────┬──────┘
                           │                    S101
        ┌──────────────────▼───────────────────────────┐
        │    ACQUIRE PULSATION DATA AND BODY            │
        │         MOVEMENT DATA                         │
        └──────────────────┬───────────────────────────┘
                           │                    S102
        ┌──────────────────▼───────────────────────────┐
        │  ESTIMATE SLEEP STAGE PROBABILITY SEQUENCE    │
        │     FROM FEATURE QUANTITY SEQUENCE            │
        └──────────────────┬───────────────────────────┘
                           │                    S103
        ┌──────────────────▼───────────────────────────┐
        │ ESTIMATE SLEEP STAGE TRANSITION PROBABILITY   │
        │  SEQUENCE FROM BODY MOVEMENT SEQUENCE         │
        └──────────────────┬───────────────────────────┘
                           │                    S104
        ┌──────────────────▼───────────────────────────┐
        │      ESTIMATE SLEEP STAGE SEQUENCE            │
        └──────────────────┬───────────────────────────┘
                           │
                    ┌──────▼──────┐
                    │     END     │
                    └─────────────┘
```

**EP 4 035 594 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011083393 A
- JP 2018161432 A
- JP 2019173326 A

### Non-patent literature cited in the description

- **PEDRO FONSECA et al.** Cardiorespiratory Sleep State Detection Using Conditional Random Fields. *IEEE Journal of biomedical and health informatics*, 01 July 2017, vol. 21 (4)